(19) Europäisches Patentamt

European Patent Office

Office européen des brevets

(11) **EP 1 046 905 A1**

(12) **EUROPEAN PATENT APPLICATION**

(43) Date of publication:
**25.10.2000 Bulletin 2000/43**

(51) Int Cl.7: **G01N 27/22**, G01N 33/487, C12M 1/34

(21) Application number: **99500057.7**

(22) Date of filing: **14.04.1999**

(84) Designated Contracting States:
**AT BE CH CY DE DK ES FI FR GB GR IE IT LI LU MC NL PT SE**
Designated Extension States:
**AL LT LV MK RO SI**

(71) Applicant: **NTE, S.A.**
**08186 Lliça d'Amunt (Barcelona) (ES)**

(72) Inventors:
- **Bragos Bardia, Ramon**
  **08071 Barcelona (ES)**
- **Lozano Nieto, Alberto**
  **08071 Barcelona (ES)**

- **Rosell Ferrer, Javier**
  **08071 Barcelona (ES)**
- **Pallas Areny, Ramon**
  **08071 Barcelona (ES)**
- **Costa Riu, Pere**
  **08071 Barcelona (ES)**
- **Elvina Canas, Jordi**
  **08014 Barcelona (ES)**

(74) Representative: **Davila Baz, Angel**
**c/o Clarke, Modet & Co.,**
**Avda. de los Encuartes 21**
**28760 Tres Cantos (Madrid) (ES)**

(54) **Method and device for measuring composition and concentration of biomass**

(57)  Method and device for measuring the concentration and composition of biomass by measuring the electrical impedance of the medium in the biomass (cells) at several frequencies and by a later adjustment of the relationship:

$$Z(f) = R_\infty + \frac{R_0 - R_\infty}{1 + \left( j \frac{f}{f_c} \right)^{1-\alpha}}$$

to experimental data. The device consists of a main electronics box (1) connected to a computer (4) and reading probes (3) each provided with electronics (2) (figure 3).

EP 1 046 905 A1

FIG. 3

**Description**

The present invention relates to a method and an device for measuring the concentration and composition of biomass which determines said characteristics by measurements of the impedance of the biomass.

Any industrial process requiring growth or control of biomass in general must determine its concentration and/or composition. Biomass measuring devices existing in the market have the disadvantage of not being reliable or precise enough, as concluded from the information presented in "Biomass Determination", Bernhard Sonnleitner, Georg Locher and Armin Fiechter, Journal of Biotechnology 26, 5-22 (1992). Normally, these devices rely on conditions which cannot be completely controlled (cell activity, existence of bubbles, etc.). The biomass measuring device required by industry must be reliable, it must depend on a physical parameter which directly provides the information on cell concentration, cannot interfere with the process undergone by the cells and must take direct readings without requiring extraction of samples from the bioreactor at certain intervals.

A system already exists which measures the impedance of the biomass in order to determine its composition. This system has the disadvantage of giving a very imprecise result due to its using only the impedance at a certain frequency and therefore using only a single parameter. This known system is even more disadvantageous when used for low-concentration biomass since in this case the signal/noise ratio is very low, often making reading impossible.

Another inconvenient of the existing systems for determining the composition of biomass is that the readings depend on the temperature and conductivity of the medium and also on the presence of bubbles in the medium.

Non-electrical known devices are disadvantageous because they measure parameters which do not have a direct relationship with the number of cells in the growth For example, measurement of the fluorescence in a growth is directly related to the metabolic activity of the cells, which under certain conditions is not directly related to cell concentration.

The object of the resent invention is therefore to provide a method and a device for measuring reliably, precisely and quickly the concentration and composition of biomass, even in low concentrations, avoiding existing problems.

The present invention achieves this by a procedure and a device which allows making several readings, at least four, of the impedance of the biomass to be investigated and in a first stage of the process the cell concentration in the growth is estimated in a general way using the following equation:

$$E(\%) = \frac{|Z(BF)| - |Z(AF)|}{|Z(BF)|} \cdot 10$$

where Z(BF) and Z(AF) are respectively the impedances at the lowest and highest frequency of the measurement sweep performed. In the second stage the following formula for the electrical impedance (Z) as a function of frequency (f) is adjusted to the impedance values measured by the least squares method:

$$Z(f) = R_\infty + \frac{R_0 - R_\infty}{1 + \left(j\frac{f}{f_c}\right)^{1-\infty}}$$

in this way determining values $R_\infty$, $R_0$, $f_c$ and a, which represent the resistance at infinite frequency, the resistance at zero frequency, the frequency at which the IM (Z) maximum occurs, which is characteristic of the type of organism of the biomass, and a parameter a related to the dispersion in cell size in the growth respectively, with which E is then calculated, defined as:

$$E(\%) = \frac{R_0 - R_\infty}{R_0} \cdot 100$$

to obtain a second general estimate of cellular concentration in the growth or biological system by the adjustment.

The present invention also provides a device for measuring concentration and composition of biomass by readings of impedance , capable of performing the aforementioned process, consisting of a main electronics box, reading probes which are connected through frontal stages to the electronics of the box, preferably by coaxial cables, means for determining the temperature of the biomass to be investigated and electronics placed in each probe.

The main electronics box is connected to a computer which generates the signal with a direct digital synthesis (DDS) circuit and consists of a demodulator, an analog-digital converter and a control and communication device. Each of the reading probes has at least four electrodes, said frontal stages containing the circuits for conditioning the measurement of injected current and the differential voltage detected.

In an example of an embodiment of the device of the present invention the probes, which are preferably bar-shaped, are provided with a transverse oblong orifice on whose longitudinal edges are placed four electrodes which are aligned by twos on opposite edges with one ring-shaped electrode and one circular electrode on each side, the electrodes on either side being placed coaxial to each other.

For biomass in thin films, placed on flat supports, the electrodes are formed by strips of metallic deposits resting on said supports, with the ends of the electrodes forming a rectangle, preferably a square.

An advantage of the present invention is that it offers greater precision in the measurement of the composition and concentration of the biomass since it determines a set of parameters resulting from an adjustment and directly related to the properties of the biomass under study.

In addition, the electrode configuration is especially adequate since on one side it limits the electrical field in a defined way, which makes it possible to measure biomass even in low concentrations, under 1 g. dry weight /l, and on the other hand it considerably reduces the probability of bubbles present in the biomass affecting the measurement.

A further advantage of the device is that it corrects the measurement of the impedance with respect to temperature and conductivity of the biomass, thus obtaining a result independent of external conditions.

Examples of an embodiment of the present invention are described in detail with the aid of drawings, where:

Figure 1 shows a spectrum of the modulus of the electrical impedance as a function of the frequency f;

Figure 2 shows a circuit diagram corresponding to the electrical model of a biological tissue;

Figure 3 shows an example of an embodiment of the device according to the present invention;

Figure 4 shows a form of embodiment of the reading probes;

Figure 5 shows a sketch of the electronic components of the device;

Figure 6 shows an example of an embodiment of the electrodes adequate for measuring biomass in film form;

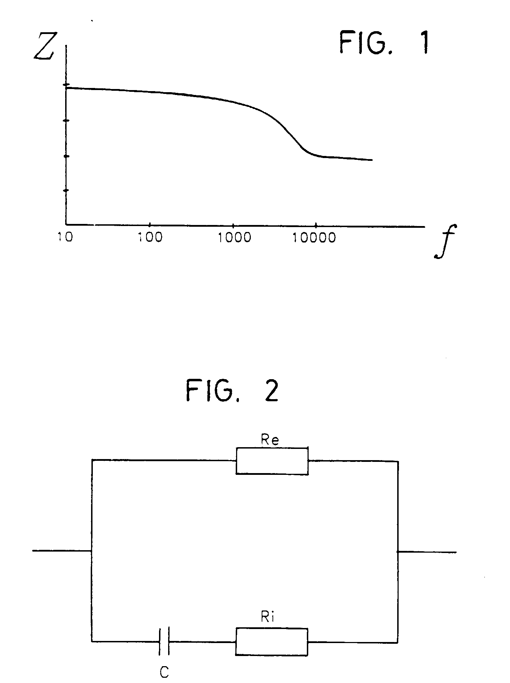

The biomass measurer is based on a physical effect known as relaxation of the electrical impedance. This effect is explained in great detail in "Dielectric Properties of Tissues and Biological Materials; A Critical Review", Foster K. and Schwan H., Critical Reviews in Biomedical Engineering, volume 17, book 1 (1989). This relaxation consists of a drop in the value of the electrical impedance with the frequency used to measure it in any given medium. The electrical impedance of a medium can be divided into a real part and an imaginary part. The real part is what is commonly known as electrical resistance and the imaginary part, for biological tissues, corresponds to the medium's electrical capacitance. The relaxation effect can be explained by the absorption of electromagnetic energy. A simple electrical model is assumed by convention, where the capacitance of the cellular membrane causes this absorption of energy. (Figure 2). The resistance of the extracellular medium is modeled by a resistance $R_E$ and the resistance of the intracellular medium by a resistance $R_i$. The capacitance of the cell membrane is modeled by a capacitor C. When the electrical impedance of a circuit such as the one shown in figure 2, a curve is obtained as the one shown in figure 1. If the injected signal is of high frequency an electrical impedance is obtained equal to the parallel resistance of the two resistances in the circuit and if it is of low frequency it is equal to the extracellular resistance.

The frequency at which the fall in impedance occurs depends on the type of cell measured in the medium. Animal cells have a lower relaxation frequency than bacteria. Therefore, the measurement of the electrical impedance at several frequencies by the device of the present invention allows determination of the type of cell present in the medium.

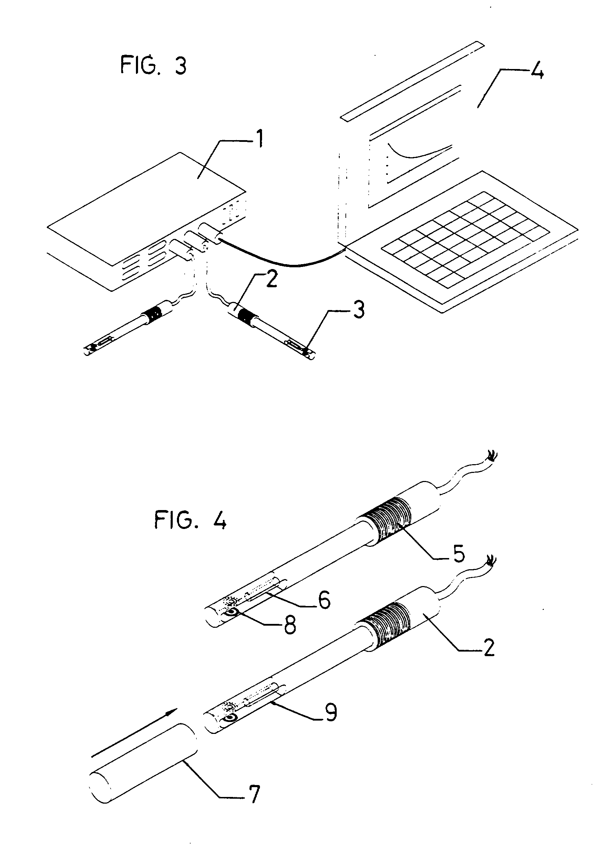

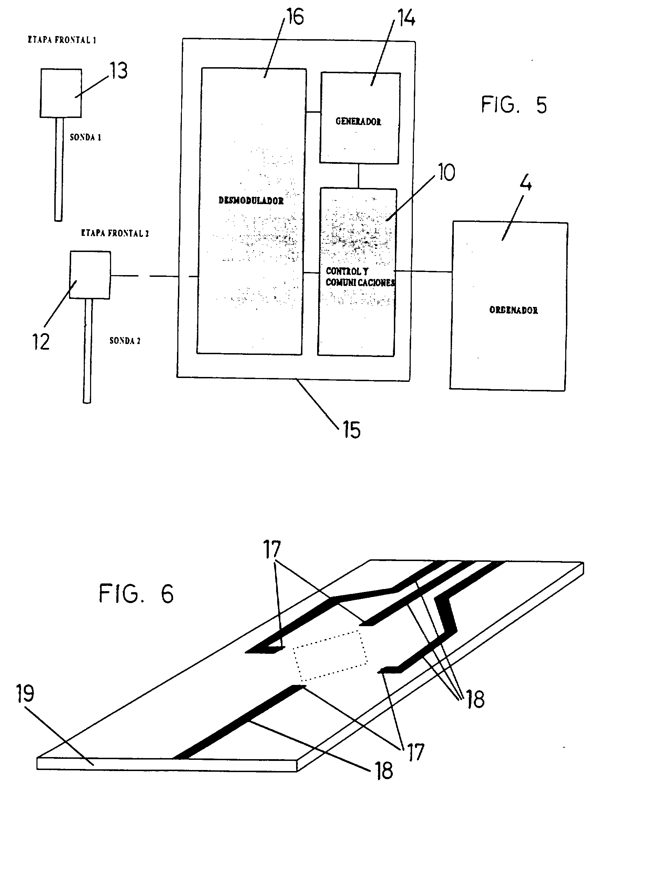

The device consists of three different elements (figure 3): the main electronics box (1), electronics in probes (2) and reading probes (3). The main box may be connected to a computer (4) although a microprocessor can be included in the electronics to replace the computer. The growth container can have any shape, although the probes will have to be different for each container shape. The device measures the electrical impedance at several frequencies in a range which can go from 1 kHz to 10 MHz, enough for all applications with biological cells, although it can be used in a wider range for general applications. In addition, it measures the temperature of the medium and the conductivity of the extracellular medium at low frequency. Probes (3) provided for the measurer can, for example, be as shown in figure 4, designed to adapt to two standard connectors of an industrial bioreactor and include a temperature gauge (6) in each probe (3), a set of four electrodes (8) in each probe contained in a capsule to avoid the entry of biological cells in the space between electrodes in the probe (3) which measures the low frequency conductivity of the medium, measurement electronics (2) which must be as close to the electrodes as possible and a standard thread(5) for an industrial bioreactor.

The electrodes employed are mixed. These are characterised by injecting current through the outer rings of the electrode set (8) and measuring the voltage across the two central discs of the set. This measurement is performed independently in each probe. The electrodes on each probe are placed by pairs on the longitudinal edges of an oblong transversal orifice.

These probes described above and shown in figure 4 are a specific application for a certain type of bioreactor. The type of probes to be employed must be studied for each container where measurements must be performed.

A fundamental factor is the confination of the electric field. The more widespread the electrical field generated by the electrodes, the more sensitive to external artifacts and the more difficult the measurement. On the contrary, a geometry which tries to confine the electric field too much may lead to very local readings which are not necessarily representative of the global properties of the biomass. Obviously a compromise must be made.

The probes shown in figure 4 allow confining the electric field between the electrodes very effectively. This type of electrode is resistant to measurement artifacts caused by bubbles outside the probe groove.

A different example of an embodiment of the probes of the present invention adequate for measurement in thin layers of growth on plates is shown in figure 6. Here metallic strips are deposited on a flat support (19) on which the growth layer is to be deposited, said metallic strips (18) being the four electrodes of the probe. The ends (17) of the electrodes form a rectangle, preferably a square, in order to limit the electric field in the area between the electrodes. The arrangement of the electrodes in a square for planar measurements is known as "Van der Pow" as has the advantage of perfectly defining the electric field.

The temperature of the medium and conductivity of the extracellular medium affect the value of the electrical impedance in a linear and calibrated manner. Therefore, measuring these values gives the measurer information on the influences other than cell growth and allows taking these into account when giving the cell concentration value.

Temperature gauge (6), which may for example consist of a thermocouple, is connected to a computer (4) which measures and performs the entire data processing before estimating the cell concentration. For fixed specific applications the computer may be replaced by a specific controller designed especially for this and placed in the electronics box (1) (Figure 3).

Materials of probes (3) are materials approved for biological use (such as Teflon, silicone or polyacetal and AISI 316 stainless steel) and must be sterilisable in order not to contaminate the growth.

The measurement process begins with the digital generation of the electrical current, although it may also be done analogically, and its injection into the growth medium by two electrodes. The other two electrodes measure the voltage drop in the medium. This is a four lead measurement which to a great extent makes the result independent of measurements of impedance of the contact point of the electrodes with the medium. The signal amplifiers are placed very near the probe to prevent parasitic capacitances from affecting the signal, since very high frequencies are used at times. The relation between the voltage signal and the electrical current signal is the electrical impedance. The process is repeated for several frequencies between 1 kHz and 20 MHz. The frequencies can be selected, the selection depending on the type of biological growth. The electrical impedance is constantly corrected with the temperature and conductivity measurement previously mentioned according to known linear coefficients.

From the data obtained two data processes may be carried out leading to the obtention of estimation parameters for biomass or cell concentration:

1) The relationship between impedance at low frequencies and the impedance at high frequencies is proportional to the concentration of cells in the medium. The measurer will be previously calibrated according to the type of cells mentioned. The relationship between the high value and the low one is a good estimation of biomass concentration:

$$E(\%) = \frac{|Z(BF)|-|Z(AF)|}{|Z(BF)|}.10$$

where $|Z(AF)|$ and $|Z(BF)|$ are the moduli of the impedances measured at the highest and lowest frequencies.

2) Measurements of the electrical impedance resulting from the frequency sweep (at least four measurements from 1 kHz to 20 MHz) are adjusted to the function which expresses the dependence of the impedance (Z) on the frequency (f):

$$Z(f) = R_\infty + \frac{R_0 - R_\infty}{1 + \left(j\frac{f}{f_c}\right)^{1-\infty}}$$

with the aid of a least squares method. Parameters $R_0$ and $R_\infty$ are the resistance of the medium in direct current and at a very high frequency respectively, f is the frequency at which the maximum imaginary part occurs which depends on the type of microorganism; y is a parameter related to the dispersion of the cell size and shape of the microorganisms.

The impedance measured can be modeled after the circuit shown in figure 2 described above. The relationships between circuit parameters ($R_e$ and $R_i$) and the mathematical model are the following:

$$R_e = R_0$$

$$R_i = \frac{R_0 \cdot R_\infty}{R_0 \cdot R_\infty}$$

where $R_0$ and $R_\infty$ are obtained from adjusting the equation modeling the variation of impedance with frequency shown previously. The relationship:

$$E(\%) = \frac{R_0 - R_\infty}{R_0}.100$$

gives an estimation of the biomass (cell) concentration. This relationship is the same one as option 1 of the data processing but with values resulting from an adjustment to the model.

In summary, the two options allow obtaining a good estimate of the biomass with the relationship between impedance at high and low frequencies. One of the options used directly employs the values measured at high and low frequency and the other uses two values resulting from adjusting the impedance data from a frequency sweep to a model.

When the second option is used, two additional parameters are obtained which give information on the cells in the system, allowing to determine the type of cell in question as well as the size dispersion. The system could also decide whether the growth is contaminated by cells of a different type.

The final data obtained by the measurer are processed by a computer or specifically designed processor and present the user with continuous data on the cell concentration in the medium.

The different components of the system are identified in figure 5, these components being:

- Frontal stages (12, 13): directly connected to the probes and containing all measurement circuits of the injected current and the differential voltage detected at the electrodes. They also contain the conditioning circuit for the temperature gauges. They are connected to the main unit by a set of leads.

- a main unit (15): it contains circuits performing the following functions:

  - generation of the signal by a direct digital synthesis (DDS) circuit which generates the sinusoidal injection and reference signals for demodulation (1 kHz- 20 MHz). Minimum 10 MHz.
  - demodulator (16) obtaining the in-phase components and quadrature of the of the current and voltage signals to determine the complex signal.
  - analogic-digital converter
  - control and communications device.

- A computer (4): runs the program for the configuration and measurement functions, as well as the presentation and storage of the results. It carries out calibration of the measurements, adjustment to the models and calculation of the biomass estimators. Calibration is a key point in this system, since very small variations in impedance are measured in the presence of important systematic errors. Three complex coefficients are used at each frequency. These coefficients are obtained from the measurement of three reference solutions.

In another example of an embodiment shown in figure 6, adequate for use with thin biomass layers placed on a flat support (19), electrodes (18) are formed by strip-shaped metallic deposits on said flat support (19). Ends (17) of said electrodes form a rectangle, preferably a square. In this way the electric field is limited to inside this square. The biomass to be measured is placed on said support (19) and on the aforementioned electrodes (18) to thus establish electrical contact between them.

The method and apparatus of the present invention may for example be used in the beer industry, where controlling yeast concentration during fermentation affects the final quality of the beer obtained.

Another field of application is growth of animal cells, essential to genetic therapy. Here growth control is crucial in order to optimise treatment time and its cost.

## Claims

1. Method for measuring the concentration and composition of biomass by using the measurement of the electrical impedance of the medium in the biomass (cells) which is suspended on it or immobilised, at several frequencies, at least four, in the range between 1 kHz and 10 MHz, these selectable frequencies depending on the type of biological growth, characterised in that in a first stage the cell concentration in the growth is estimated using the following equation:

$$E(\%) = \frac{|Z(BF)| - |Z(AF)|}{|Z(BF)|} \cdot 10$$

where Z(BF) and Z(AF) are respectively the impedances at the lowest and highest frequency of the measurements sweep performed;
and in a second stage the following relationship for electrical impedance is adjusted to the measured impedance values by a least squares method:

$$Z(f) = R_\infty + \frac{R_0 - R_\infty}{1 + \left(j\frac{f}{f_c}\right)^{1-\infty}}$$

in this way determining values $R_\infty$, $R_0$, $f_c$ and a, which stand for the resistance at infinite frequency, the resistance at zero frequency, the frequency at which the maximum is obtained for Im(Z), which is characteristic of the type of microorganism of the biomass, and a parameter related to the dispersion of cell size in the growth respectively, with which E is then calculated, defined as:

$$E(\%) = \frac{R_0 - R_\infty}{R_0} \cdot 100$$

in order to obtain a second general estimate of the cell concentration in the growth or biological system by the adjustment.

2. Procedure as claimed in claim 1, characterised in that the impedance is constantly corrected with the temperature and conductivity measurement of the medium.

3. Device for measuring the concentration and composition of biomass by measurements of the impedance, capable of performing the process as claimed in claims 1 and 2, characterised in that it consists of a main electronics box (1) connected to a computer (4) which generates the signal by a direct digital synthesis (DDS) circuit and is provided with a demodulator (16), an analog-digital converter and a control and communication device (10); reading probes (3) which are connected by front stages (12, 13) to the electronics of main box (1), preferably by coaxial leads, of which at least one has four electrodes (8), these frontal stages (12, 13) having the conditioning circuits for measurement of injected current and differential voltage read; means for determining the temperature of the biomass whose concentration is to be determined and electronics (2) in each probe.

4. Device as claimed in claim 3, characterised in that the main electronics box (1) includes a microprocessor instead of the computer (4).

5. Device as claimed in claims 3 and 4, characterised in that probes (3),preferably bar-shaped, have a transverse oblong orifice (9) on whose longitudinal edges are four electrodes (9) arranged in pairs aligned on opposite edges, one electrode on each side being ring-shaped and the other being circular, electrodes on each side being placed coaxial to each other.

6. Device as claimed in claims 3 and 4, characterised in that for biomass in thin films placed on a flat support (19), electrodes (10) are formed by strip-shaped metallic deposits on said flat support, the ends (12) of these electrodes forming a rectangle, preferably a square.

FIG. 1

FIG. 2

FIG. 3

FIG. 4

ETAPA FRONTAL 1

13

SONDA 1

16

14

FIG. 5

GENERADOR

DESMODULADOR

ETAPA FRONTAL 2

10

4

12

SONDA 2

CONTROL Y
COMUNICACIONES

ORDENADOR

15

FIG. 6

17

19

18

17

18

**European Patent Office**

**EUROPEAN SEARCH REPORT**

Application Number

EP 99 50 0057

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| Y | EP 0 865 763 A (NTE S A) 23 September 1998 (1998-09-23) * column 11, line 23-55; claims 1,3 * | 1-6 | G01N27/22 G01N33/487 C12M1/34 |
| Y | WO 93 14402 A (ABER INSTR LTD) 22 July 1993 (1993-07-22) * page 7, line 8 - page 9, line 11; claim 17; figures 1,2 * | 1-6 | |
| Y | EP 0 689 051 A (MATSUSHITA ELECTRIC IND CO LTD) 27 December 1995 (1995-12-27) * column 5, line 50,51; claims 5-7; figure 3 * | 6 | |
| A | WO 88 02115 A (KELL DOUGLAS BRUCE ;TODD ROBERT WILLIAM (GB)) 24 March 1988 (1988-03-24) * claim 1 * | 3 | |
| A | GB 2 329 711 A (UNIV WALES ABERYSTWYTH THE) 31 March 1999 (1999-03-31) * page 1-4 * | 1,3 | TECHNICAL FIELDS SEARCHED (Int.Cl.7) |
| A | EP 0 277 789 A (KOBE STEEL LTD) 10 August 1988 (1988-08-10) * abstract; claim 8 * | 1,3 | G01N C12M |
| A | WO 92 16835 A (TRAPPL HELMUT) 1 October 1992 (1992-10-01) * claim 1 * | 3 | |
| A | EP 0 869 360 A (NTE S A) 7 October 1998 (1998-10-07) * column 5, line 19-22; claim 1 * | 2 | |

The present search report has been drawn up for all claims

| Place of search | Date of completion of the search | Examiner |
|---|---|---|
| BERLIN | 20 September 1999 | Brison, O |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**  EP 99 50 0057

This annex lists the patent family members relating to the patent documents cited in the above-mentioned European search report.
The members are as contained in the European Patent Office EDP file on
The European Patent Office is in no way liable for these particulars which are merely given for the purpose of information.

20-09-1999

| Patent document cited in search report | | Publication date | Patent family member(s) | | Publication date |
|---|---|---|---|---|---|
| EP 0865763 | A | 23-09-1998 | NONE | | |
| WO 9314402 | A | 22-07-1993 | AU | 658971 B | 04-05-1995 |
| | | | AU | 3261393 A | 03-08-1993 |
| | | | DE | 69301910 D | 25-04-1996 |
| | | | DE | 69301910 T | 05-09-1996 |
| | | | EP | 0620919 A | 26-10-1994 |
| | | | GB | 2280271 A,B | 25-01-1995 |
| | | | JP | 7502660 T | 23-03-1995 |
| | | | US | 5551281 A | 03-09-1996 |
| EP 0689051 | A | 27-12-1995 | CN | 1131744 A | 25-09-1996 |
| | | | JP | 8062209 A | 08-03-1996 |
| | | | KR | 150390 B | 01-10-1998 |
| | | | US | 5563067 A | 08-10-1996 |
| WO 8802115 | A | 24-03-1988 | AT | 62071 T | 15-04-1991 |
| | | | AU | 593387 B | 08-02-1990 |
| | | | AU | 7881087 A | 24-03-1988 |
| | | | CA | 1261393 A | 26-09-1989 |
| | | | EP | 0281602 A | 14-09-1988 |
| | | | NZ | 221878 A | 27-09-1989 |
| | | | US | 4810650 A | 07-03-1989 |
| | | | ZA | 8706972 A | 22-03-1988 |
| GB 2329711 | A | 31-03-1999 | WO | 9917124 A | 08-04-1999 |
| EP 0277789 | A | 10-08-1988 | JP | 2117395 C | 06-12-1996 |
| | | | JP | 7117511 B | 18-12-1995 |
| | | | JP | 63191046 A | 08-08-1988 |
| | | | JP | 1067200 A | 13-03-1989 |
| | | | JP | 1884829 C | 10-11-1994 |
| | | | JP | 6009519 B | 09-02-1994 |
| | | | JP | 1124399 A | 17-05-1989 |
| | | | JP | 1736742 C | 26-02-1993 |
| | | | JP | 4025800 B | 01-05-1992 |
| | | | AT | 76436 T | 15-06-1992 |
| | | | DE | 3871168 A | 25-06-1992 |
| | | | US | 5182193 A | 26-01-1993 |
| WO 9216835 | A | 01-10-1992 | EP | 0529050 A | 03-03-1993 |
| EP 0869360 | A | 07-10-1998 | NONE | | |

EPO FORM P0459

For more details about this annex : see Official Journal of the European Patent Office, No. 12/82